# EUROPEAN PATENT APPLICATION

(11) **EP 4 226 923 A1**
(43) Date of publication of application: **16.08.2023**
(21) Application number: 20964396.4
(22) Date of filing: 04.12.2020
(51) Int. Cl.: A61K 31/435, A61K 31/192, A61P 11/00

(54) **PHARMACEUTICAL COMPOSITION HAVING AN ANALGAESIC AND AN ANTIHISTAMINE FOR TREATING RESPIRATORY DISEASES**

(71) Applicant: Laboratorios Silanes, S.A. de C.V., Mexico, 11000 (MX)
(72) Inventor: GONZÁLEZ CANUDAS, Jorge Alejandro, Ciudad de México, 11000 (MX); MUÑOZ MARTÍNEZ, Cecilia Jannette, Ciudad de México, 11000 (MX); OLLERVIDES RUBIO, Paola Yazmín, Ciudad de México, 11000 (MX); ESPINOZA LEÓN, Sixto Serafín, Ciudad de México, 11000 (MX); MÁRQUEZ MEJÍA, Jannette, Ciudad de México, 11000 (MX)
(74) Representative: Araujo, Daniel
(86) International application number: PCT/MX2020/050051
(87) International publication number: WO 2022/119431

(57) **Abstract**

This invention involves an improved, immediate-release pharmaceutical composition against respiratory diseases and acute upper respiratory tract infections (IAVRS) with a stable bioavailability and comprises an analgesic, such as ibuprofen; an antihistamine, such as loratadine; and one or more excipients, such as suspending agents, solubilizers, co-solvents, sweeteners or flavorings, colorants, preservatives or antimicrobials, and solvents. The manufacturing process of the composition manages to overcome the technological complexity of having both drugs in liquid form while preserving their physicochemical properties.

## Description

### FIELD OF THE INVENTION

This invention relates to the field of pharmaceutical and medicinal chemistry, in particular, to combination pharmaceutical compositions for the treatment and control of respiratory diseases.

### BACKGROUND

An acute upper respiratory tract infection (IAVRS, by its Spanish acronym) refers to an infectious disease that affects the respiratory system from the nose until before the epiglottis for a period of less than 15 days and that is frequently caused by viruses and occasionally by bacteria.

Acute upper respiratory tract infections are the leading cause of illness in Mexico; it is also the primary reason why medical attention is sought. The disease occurs in all age groups; however, two-thirds of the cases observed in the general population occur in pediatric patients, and approximately one-third occur in children under 4 years of age.

Children have between 2 and 4 episodes of respiratory infection annually; however, it is not uncommon for them to have 5 to 8 episodes of common cold a year.

In 80 to 90%m the etiology is viral (ENSANUT 2012); these episodes of infection are generally benign and self-limiting in a short time.

In a smaller proportion, between 15 and 30% of cases in children and between 5 and 20% in adults, the etiology is bacterial: *Streptococcus pyogenes* (group A hemolytic Streptococcus β EBHA), *Streptococcus Pneumoniae, Mycoplasma pneumoniae, Neisseria meningitidis,* and *Neisseria gonorrhoeae.* Based on the above, it can be stated that most patients with IAVRS require only symptomatic treatment.

This nosological group of IAVRS includes:
- Acute Otitis Media
- Tonsillitis and pharyngotonsillitis
- Adenoiditis
- Rhinopharyngitis or common cold
- Allergic rhinitis

Otitis media is characterized by pain, fever, otorrhea, or prominent tympanic membrane. The first recommendation for treatment is to use ibuprofen or acetaminophen, both of which have evidence of effectiveness.

Acute pharyngo tonsillitis (FAA) is defined as an acute febrile process of generally infectious origin that occurs with inflammation of the pharynx mucous membranes and/or pharyngeal tonsils, in which the presence of erythema, edema, exudates, ulcers, or vesicles can be objectified. The most common etiology is viral. At any age, the presence of conjunctivitis, rhinorrhea, aphonia, cough, and/or diarrhea is a finding suggestive of a viral origin of the infection.

Rhinoviruses are usually the cause of the common cold. Other agents are coronaviruses, respiratory syncytial virus, influenza virus, and adenovirus. Common cold is transmitted hand to hand. Mast cells and T and B lymphocytes increase in the nasal mucosa of patients with rhinopharyngitis. In addition, nasal eosinophilia is prolonged in allergic patients. Upper respiratory infections can cause otitis media, sinusitis, exacerbate asthma, and cause lower respiratory tract infections in neonates, older adults, and immunocompromised patients. This results in an increased use of medical resources including emergency room visits and hospitalizations.

Approximately one-fifth of children with rhinopharyngitis receive treatment that is contraindicated for their age. The appropriate treatment for this pathology is second-generation antihistamines, such as loratadine, which only affects histamine receptors and does not cross the blood-brain barrier and, therefore, does not cause drowsiness. Combining treatment with NSAIDs such as ibuprofen significantly reduces headache, otalgia, sneezing, myalgias, arthralgias, and fever associated with rhinopharyngitis.

Allergic rhinitis in particular is characterized by symptoms such as pruritus in the eyes, nose, palate, and ears, as well as watery rhinorrhea, sneezing, nasal congestion, dripping, or postnasal drainage. Allergic rhinitis is a disease with marked prevalence that affects approximately 20% of the adult population of the United States and even 40% of children. It can have health and productivity consequences if not treated properly. The burden of this disease lies not only in its negative effect on the physical and social functioning of the patient, but also in its financial burden.

Allergic rhinitis can be a causative factor of comorbid diseases such as asthma, otitis media with effusion, sleep disorders, adenoid hypertrophy, and sinusitis. Allergic rhinitis results in approximately $16.7 million in physician visits each year and its treatment with uncontrolled medications and medical prescriptions results in direct costs of approximately $4.5 billion annually in the United States. In addition, an estimated $3.8 million of absenteeism from work and school.

Allergic rhinitis is the leading cause of allergy consultation in many countries including Mexico and one of the top ten in primary care. The global prevalence of allergic rhinitis has increased in recent decades. The International Study of Asthma and Allergies in Childhood, ISAAC, Phase III reports a total estimated prevalence in Mexico of 4.6%.

The characteristics suggestive of acute respiratory infection of viral etiology are:
- Conjunctivitis
- Coryza
- Cough
- Hoarse voice
- Stomatitis
- Exanthema

The common cold is a self-limiting illness that usually lasts 5 to 14 days and is characterized by a combination of some of the following symptoms:
- Rhinorrhoea
- Cough
- Fever
- Odynophagia
- General discomfort
- Decrease in appetite
- Cephalea
- Irritability

On the other hand, nonsteroidal anti-inflammatory drugs (NSAIDs) are widely administered as part of pain therapies. NSAIDs are a group of compounds among which aspirin, celecoxib, diclofenac, ketoprofen, ketorolac, ibuprofen, and others stand out. NSAIDs are widely used in upper airway infections, as they decrease headache, myalgias, arthralgias, and improve general condition. They are commonly used in treatments for pharyngitis and tonsillitis, otitis media, sinusitis, among other respiratory diseases.

Ibuprofen is a derivative of propionic acid and has anti analgesic, anti-inflammatory, and antipyretic properties; in addition it inhibits the activity of cyclooxygenase I and II, resulting in a decrease in the formation of precursors of prostaglandins and thromboxanes. The therapeutic effect of ibuprofen derives from the inhibitory activity of the prostaglandin synthase. Its chemical name is (±)-2-(4-isobutylphenyl) propionic acid.

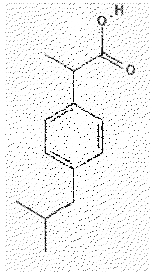

Ibuprofen is widely used to reduce fever and for the treatment of pain or inflammation caused by various conditions such as headache, dental pain, back pain, arthritis, and menstrual cramps, among others. It is also used to relieve minor aches and pains due to the common cold or flu potentially benefiting the treatment of viral respiratory infections (Winther, 2003). However, the result is inconclusive and requires confirmatory studies: Ibuprofen has also been studied in combination with other drug products in an experimental setting with volunteers inoculated with rhinovirus. Additional symptomatic benefits were found when ibuprofen (400 mg) was combined with pseudoephedrine (60 mg) (Sperber et al., 1989). This combination tended to reduce the cumulative nasal symptom score (days 1 to 6) compared to pseudoephedrine alone. Llor et *al.,* conducted a study to evaluate the efficacy of oral ibuprofen or amoxicillin compared to placebo in improving cough in patients with uncomplicated acute bronchitis, concluding that the median number of days with cough was lower in numbers, but not statistically significant, in the ibuprofen group (9 days; 95% Cl: 8-10 days), compared to participants who received antibiotics (11 days; 95% Cl: 10-12 days) or placebo (11 days; 95% Cl: 8-14 days). Adverse events were more common in the antibiotic group (12%), compared to ibuprofen or placebo (5% and 3%, respectively, P = 0.008). (Llor, 2013). Based on this evidence, the use of ibuprofen may be recommended at the onset of the first symptoms of an airway infection prior to the initiation of antibiotics.

NSAIDs in combination with antihistamines have also been shown to have a positive interaction in relieving cold or flu symptoms. Antihistamines are widely used to treat allergies. There is a wide variety of antihistamines, although not all are effective, and some can have major side effects. For example, combination therapy for the treatment of rhinopharyngitis is one of the best strategies to reduce the associated symptoms and therefore reduce lost work and school days. Several clinical studies have been conducted to demonstrate this.

For example, Gwaltney conducted a randomized, double-blind clinical trial evaluating the effect of ibuprofen plus a first-generation antihistamine and an intranasal interferon, finding that rhinopharyngitis symptoms decreased by 33 to 73 percent. Taking into account that in this study the author used a first-generation antihistamine that has effects on the central nervous system (CNS), such as drowsiness, the fact of having a product that integrates a second generation antihistamine that has a limited or almost no action on the CNS will be a good alternative for the treating physician.

On the other hand, publication WO1992015332 claims a non-steroidal anti-inflammatory (NSAID) which may be selected from the group meclofenamic acid, salicylic acid, ibuprofen, naproxen, diclofenac, and mixtures thereof, combined with an antihistamine selected from the group, diphenhydramine, pseudophedrine, ranitidine, loratadine, cimetidine, hismanal, terfenadine, and mixtures thereof, in a 1:1 ratio, preferably indicated for meclofenamic acid and a sedative antihistamine diphenhydramine.

Publication US4552899 describes a tablet composition of an NSAID with a sedative antihistamine. The NSAID is selected from ibuprofen, naproxen, flurbiprofen, fenoprofen, ketoprofen, suprofen, fenbufen, and fluprofen; the sedative antihistamine is phenylpropanolamine, or pseudoephedrine.

Sedative antihistamines, also known as first-generation antihistamines, have many undesirable side effects, such as sedation, drowsiness, and increased appetite, among others, which has increased the use of second-generation or non-sedating antihistamines, which have minor side effects.

Patent US4783465 discloses a composition of ibuprofen or Naproxen with an indicated non-sedating antihistamine in capsule form, wherein the non-sedating antihistamine is selected from AHR-11325, astemizole, azatadine, azelastine, cetirizine, ebastine, ketotifen, lodoxamide, loratidine, levocabastine, mequitazine, oxatomide, setastine, taxifoline, temelastine, and terfenadine, but preferably terfenadine, which is used to treat respiratory conditions. However, terfenadine has been associated with cardiac arrhythmias, so its use has been restricted and in some countries it was withdrawn from the market. (Essential Medicines and Health Products Information Portal, by World Health Organization, 2001)

In particular, loratadine is a long-acting antihistamine that has high selectivity for peripheral histamine H1 receptors and lacks the central nervous system depressant effects often associated with some of the first-generation antihistamines.

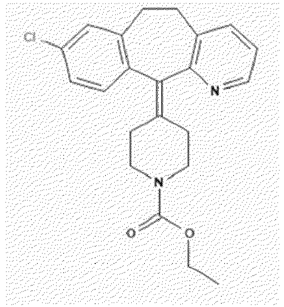

Some uses of loratadine are in the treatment of symptoms of allergic rhinitis, especially symptoms such as sneezing, tearing, and rhinorrhea. It is indicated in chronic non-specific urticaria. It is also indicated in allergic conjunctivitis.

Patent MX180700 protects an extended-release coated tablet from a combination of ibuprofen, an antihistamine loratadine, and pseudoephedrine, where ibuprofen and loratadine are not together and are prepared in separate mixtures. Ibuprofen is mixed with pseudoephedrine to the core of the tablet, and loratadine is mixed in the coating.

Patent CN202537982 describes a controlled release tablet composition; each tablet contains 5 mg of loratadine, 500 mg of ibuprofen, and 120 mg of pseudoephedrine sulfate, wherein loratadine is in the coating layer and ibuprofen and pseudoephedrine, in the core portion.

However, patents MX180700 and CN202537982 do not solve the problem of combining ibuprofen and loratadine in a single pharmaceutical composition; therefore, ibuprofen and antihistamine are kept in separate layers. Furthermore, to achieve the desired effect, it resorts to a third antihistamine that increases the side effects. Likewise, it does not solve how to formulate both drugs in the composition of better oral administration, as in suspension, which presents advantages over solid formulations such as achieving a therapeutic effect in a shorter time compared to solid dosage forms.

In view of the above, Laboratorios Silanes, concerned about the health of the population, proposes in this invention a pharmaceutical composition comprising ibuprofen and loratadine, a second-generation antihistamine, against respiratory diseases and acute infections of the upper respiratory tract (IAVRS), as well as for its symptomatic treatment in a liquid pharmaceutical form or stable suspension.

### SUMMARY OF THE INVENTION

This invention involves pharmaceutical compositions derived from a single dose unit against respiratory diseases that comprises a pharmaceutically and therapeutically acceptable amount of: a) an analgesic in a concentration of 8 g per 100 mL or an equivalent concentration; b) an antihistamine in a concentration of 0.1 g per 100 mL or an equivalent concentration; c) and one or more pharmaceutically acceptable excipients. This composition is of immediate release and improved stability. The analgesic is selected from an ibuprofen base, racemic, or enantiomers thereof, and pharmaceutically acceptable salts thereof, while the antihistamine is selected from loratadine or a pharmaceutically acceptable salt thereof. Pharmaceutically acceptable excipients are selected from one or more suspending agents, one or more solubilizers, one or more cosolvents, one or more sweeteners or flavorings, one or more colorants, one or more preservatives or antimicrobial agents, and one or more solvents.

The pharmaceutical composition is adapted to be administered orally in the form of a solution, suspension, emulsion, syrup, and/or elixir. In one form of this invention, the pharmaceutical composition of ibuprofen and loratadine is in the form of an oral suspension, promoting its administration.

In another form of the invention, the composition comprises a concentration of 400 mg of ibuprofen in 5 mL and a concentration of 5 mg of loratadine in 5 mL in a single dose unit.

Another aspect of the invention is that the process of the composition manages to overcome the technological complexity that lies in having both drugs in liquid form, preserving the physicochemical properties of the actives, as well as conferring organoleptic properties to the final product that guarantee acceptance by the patient.

The pharmaceutical composition for the manufacture of a drug product is useful in the treatment and control of respiratory diseases and acute upper respiratory tract infections (IAVRS) such as acute otitis media, tonsillitis and pharyngogillitis, adenoiditis, rhinopharyngitis or common cold, andallergic rhinitis.

### DETAILED DESCRIPTION

### Definitions

Pharmaceutically acceptable salt: The term "pharmaceutically acceptable salt" of a given compound refers to salts that retain the biological efficacy and properties of said compound, and that are not biologically, or otherwise, undesirable (P. Heinrich Stahl and Camille G. Wermuth (Eds.) Pharmaceutical Salts Properties, Selection, and Use (International Union of Pure and Applied Chemistry), Wiley-VCH; 2nd Revised Edition (May 16, 2011)]. Pharmaceutically acceptable base addition salts can be prepared from inorganic or organic bases. Salts derived from inorganic bases include, by way of example only, sodium, potassium, lithium, ammonium, calcium, and magnesium salts. Salts derived from organic bases include, but are not limited to, salts of primary, secondary, and tertiary amines. Specific examples of suitable amines include but are not limited to isopropylamine, trimethylamine, diethylamine, tri (isopropyl) amine, tri (n-propyl) amine, ethanolamine, 2-dimethylaminoethanol, tromethamine, lysine, arginine, histidine, caffeine, procaine, hydrabamine, choline, betaine, ethylenediamine, glucosamine, N-alkylglucamines, theobromine, purines, piperazine, piperidine, morpholine, N-ethylpiperidine, and the like.

Pharmaceutically acceptable acid addition salts can be prepared from inorganic or organic acids. Salts derived from inorganic acids include hydrochloric acid, hydrobromic acid, sulfuric acid, nitric acid, phosphoric acid, and the like. Salts derived from organic acids include acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, methanesulfonic acid, ethanesulfonic acid, p-toluene-sulfonic acid, salicylic acid, and the like.

Excipient: it is an ingredient of the present pharmaceutical composition that includes diluents, disintegrants, lubricants, coating systems, adsorbents, among others.

Stability: it is the ability of a pharmaceutical product to retain its chemical, physical, microbiological, and biopharmaceutical properties within specified limits throughout its shelf life.

This invention relates to pharmaceutical compositions against respiratory diseases; these immediate-release compositions are stable and have an improved bioavailability. It also relates to a suspension formulation that improves the form of administration.

The immediate release pharmaceutical composition of this invention comprises, in a single dosage unit, a pharmaceutically and therapeutically acceptable amount of:
a) an analgesic in a concentration of 8 g per 100 ml or an equivalent concentration,
b) an antihistaminic in a concentration of 0.1 g per 100 ml or an equivalent concentration,
c) and one or more pharmaceutically acceptable excipients.

The analgesic is selected from any pharmaceutically acceptable form of ibuprofen, including but not limited to ibuprofen base, racemic ibuprofen or its enantiomers, and pharmaceutically acceptable salts thereof. The antihistamine is selected from loratadine, a second-generation antihistamine, or a pharmaceutically acceptable salt thereof. Second-generation antihistamines are more selective for peripheral H1 receptors and do not cross the blood-brain barrier, because radicals that make these compounds less lipophilic have been added or removed in their molecular structure. This means that they have a different adverse effect profile and produce less sedation and anticholinergic effects.

In a preferred form, ibuprofen is in an equivalent concentration of 400 mg in 5 mL. In another preferred form, loratadine is in an amount of 5 mg in 5 mL.

The formulation of this invention includes pharmaceutically acceptable excipients that confer unique physicochemical properties to it. Excipients are selected, among other sources, from one or more suspending agents, one or more solubilizers, one or more cosolvents, one or more sweeteners or flavorings, one or more colorants, one or more preservatives or antimicrobial agents, and one or more solvents.

Pharmaceutically acceptable suspending agents are selected from carboxymethylcellulose, hydroxypropyl methylcellulose, hydroxypropylcellulose, xanthan gum, tragacanth, bentonite, methylcellulose, polyethylene glycols, microcrystalline cellulose and carbomer, or a combination thereof, among others. The preferred suspending agent for this invention is xanthan gum. The suspending agent may be present in concentrations of less than 1%. The suspending agent is preferably in a concentration of 0.42%.

Pharmaceutically acceptable antimicrobial agents or preservatives are selected from ethanol, propanol, betaines, benzalkonium chloride, benzethonium chloride, sodium benzoate, lauric arginate, methylbenzethonium chloride, cetylpyridinium chloride, 2,4,4'-trichloro-2-hydroxydiphenyl ether (Triclosan), propylparaben, methylparaben, sodium propylparaben, sodium methylparaben, hydroxybutylanisole, chlorhexidine isethionate, chlorhexidine hydrochloride, hexetidine, Quaternium 15, trichlorocarbon, polyhexamethylenebiguanide, cetylpyridinium chloride, imidazolidinylurea, diazolidinylurea, 3-lodo-2-propynyl N-butylcarbamate, 2-methyl-4-isothiazolin-3-one, dimethyldichlorohydricanthine (5-chlorophurene), phenol, or a combination thereof, among others. The preferred preservative for this invention is sodium benzoate. The preservative may be present in concentrations less than 1%.

Pharmaceutically acceptable solubilizers are selected from ethanol, water, sorbitol, glycerin, polyglycols such as polyethylene glycol (degrees 200 to 6000), polyvinylpyrrolidone (degrees 25, 30, 60, 0 90), polysorbate 20 or polysorbate 80, poloxamer, poloxamer 188, gum arabic, sodium lauryl sulfate, and sodium dioctyl sulfosuccinate, among others, in a concentration greater than 5%. The solubilizer is preferably in a concentration of 6.94%.

Dispersants may also be incorporated. The preferred dispersant of this invention is poloxamer. The dispersant may be present in concentrations greater than 5%.

Additionally, one or more co-solvents selected from ethanol, propylene glycol, glycerin, cremophor, polyethylene glycol, polyethylene glycol 300 or 400, and glycerin or a combination thereof are used, among others. The co-solvents may be present in a preferred concentration of 20% polyethylene glycol and 60% glycerin.

The sweetening, flavoring, and aroma agents are selected from sucralose, sucrose or fructose, aspartame or acesulfame potassium, and sorbitol or a combination thereof, among others. The preferred sweetener of this invention is sucralose. The sweetener may be present in a concentration of 0.42%. Consumption should not exceed 15 mg/kg body weight per day.

Pharmaceutically acceptable dyes include, but are not limited to, No. 10 yellow color, No. 5 yellow color, No. 6 yellow color, CM-208 medium red color, No. 40 red color, or the like. Preferably, the dye is red color No. 40.

In various publications, reference is made to the combination of NSAIDS with antihistamines in solid dosage form. Said dosage form has the advantage that solid state drugs have greater stability, so the main technological challenge is to ensure compatibility and ensure rheological properties that allow manufacturing and therefore compliance with specifications.

A liquid dosage form has the advantage of achieving a therapeutic effect in a shorter time compared to solid dosage forms since it does not require the break-up of the solid particles to give way to the dissolution of the drugs and consequently to their absorption. An additional advantage is that administration to the paediatric sector is facilitated since swallowing is not required to achieve its intake. It was decided to develop this project considering the above-mentioned advantages.

The technological challenges presented by liquid dosage forms are that, in the case of suspensions, as the system is heterodispersed, the drugs tend to be more unstable and a higher dose of Ibuprofen is required; in addition, masking the bitter taste of the drugs is difficult. These technological challenges were overcome in this proposal, which were not obvious enough to be overcome in the previous state of the art.

Accordingly, in one form of the invention, the pharmaceutical composition is adapted to be administered orally, preferably in liquid dosage form, therefore ensuring a better acceptance by the patient. The technological challenge was to preserve and maintain the physicochemical properties of the drug substance, as well as to confer organoleptic properties to the final product.

The pharmaceutical composition may be in the dosage form of a solution, suspension, emulsion, syrup, and/or elixir, or any other pharmaceutically acceptable liquid form. The preferred dosage form of this invention is a suspension, since it is a dosage form that can be administered in different populations, and that allows to adjust the dose based on the medical requirement. This dosage form is widely accepted for its easy administration.

This invention relates to the pharmaceutical composition as the manufacturing method for obtaining it. The method allows to obtain a pharmaceutical composition of easy administration in a single dose.

The technological complexity lies in the fact that, when both drugs are present under heat and light in a medium with a high-water content, their stability and compliance with chemical parameters must first be guaranteed. The appropriate selection of excipients and manufacturing conditions plays a very important role in the development of pharmaceutical compositions, in relation to the release of the drug and the rate of absorption in the body. The choice of excipients and the percentages thereof are vital to achieve the required bioavailability profile for both drugs, ibuprofen, and loratadine.

For another form of the invention, the production process of the tablets was based on the selection of unit operations, order, and execution time to control the different physicochemical properties of the drugs.

The pharmaceutical composition of this invention can be used for the manufacture of a drug product that is useful in the treatment and control of respiratory diseases and acute upper respiratory tract infections (IAVRS). The IAVRSs are selected from acute otitis media, tonsillitis and pharyngogillitis, adenoiditis, rhinopharyngitis or common cold, or allergic rhinitis.

Due to the innovative approach applied to the composition process and formulation, it was possible to obtain a product with demonstrated stability through studies in accordance with current regulations in which the quality attributes were met during the evaluation period. The product is stable under the temperature and humidity conditions established in the country, as stated in NOM-073. Stability testing is a means of comparing different formulations, packaging materials, or manufacturing processes in short-lived experiments.

The initial and accelerated results for the determination of the stability of the compositions of this invention for the manufacture of a drug product useful in the treatment and control of respiratory diseases and acute upper respiratory tract infections (IAVRS) are shown below in Example 4.

Technological development comprised a series of evaluations defining the formulation and process as described below. Experts in the art will find that multiple variations and modalities can be applied in the development of this invention without turning away from its spirit and scope to ensure the proper functioning of the product and meet the required quality characteristics.

### Examples

### Example 1. Manufacturing process for the preparation of the pharmaceutical composition

The following pages present the manufacturing process for the pharmaceutical composition in suspension form of this invention, which comprises, among other components, ibuprofen base, racemic ibuprofen or its enantiomers, and pharmaceutically acceptable salts thereof in combination with loratadine or some pharmaceutically acceptable salts thereof.
1. Mix 25% of co-solvent 1, 33% of co-solvent 2, 67% of solubilizer, and drug 2 for no less than 20 minutes (Dispersion 1)
2. Mix 23% of solvent, 100% of sweetener, 100% of flavor, and 100% of preservative 2 for no less than 10 minutes (Mixture 2)
3. Mix 3% of co-solvent 2 and 100% of suspending agent for 5 minutes (Dispersion 2)
4. Add Dispersion 2 to Mixture 2 and mix for 10 minutes (Mixture 3)
5. Mix 33% of the solubilizer, 75% of co-solvent 1, and drug 1 for no less than 15 minutes (Dispersion 3)
6. Add 3.64% of co-solvent 2 to the dispersion and mix for 10 minutes (Dispersion 4).
7. Mix dispersion 4 with mixture 3 for 15 minutes (Mixture 4).
8. Add dispersion 1 to mixture 4 while stirring for not less than 15 minutes (Mixture 5).
9. Mix 0.6% of the solvent with the dye for 5 minutes (Mixture 6).
10. Incorporate mixture 6 into mixture 5 and stir for not less than 10 minutes (Mixture 7).
11. Dilute the mixture 7 to the desired volume
12. Homogenize the previously diluted mixture.

### Example 2. Qualitative-quantitative formula of the pharmaceutical composition

A series of tests were performed. The formulation components were selected based on their function and the concentration was established in relation to the behavior of the product, ensuring its proper functioning.

**Table 1. Formulation of the pharmaceutical composition**

| g/100 mL | Component | Function |
|---|---|---|
| 8.00 | ibuprofen | Drug 1 |
| 0.100 | loratadine | Drug 2 |
| 0.500 | Xantana rubber | Suspending agent |
| 5.00 | Poloxamere 188 | Solubilizer |
| 15.00 | Polyethilene Glycol 400 | Co-solvent 1 |
| 43.00 | Glycerine | Co-solvent 2 |
| 0.30 | Sucralose | Sweetener |
| 0.30 | Sodium benzoate | Preservatives |
| 0.075 | Fruits of the forest flavor | Flavorings |
| 0.002 | Color Red No. 40 | Dye |
| 100.00 | Purified Water c.b.p (mL) | Solvent |

### Example 3 Bioavailability tests

A non-pharmacokinetic interaction study was performed with the combination of 800 mg/10mg ibuprofen/loratadine suspension, administered individually, in combination, or in a single dose in healthy subjects of both genders under fasting conditions. The objective of this study was to compare the pharmacokinetic profile (Cₘₐₓ and AUC_{t 0}), of the combination 800 mg/10 mg/10 mL ibuprofen/loratadine suspension, single dose, versus each component administered individually (800 mg of ibuprofen and 10 mg of loratadine) to establish the non-interaction of the combined drugs.

This study allowed the determination of the bioavailability of 800 mg ibuprofen /10 mg loratadine administered orally in combination or individually in a single dose in clinically healthy subjects based on NOM 177-SSA1-2013.

This was a prospective, open-label, longitudinal, single-dose crossover study of the 800 mg ibuprofen/10 mg /10 mL loratadine suspension combination administered orally versus each component administered individually with three treatments, three periods, six sequences, a 1-week (7-day) washout period, and a total of 72 healthy subjects of both genders under fasting conditions. Blood samples were taken from each subject in each of the 3 periods at 18 different times in 24 h.

The analytical method for the plasmatic quantification of ibuprofen and loratdine was based on the methods published by Song H. *et al.* and Vlase L. *et al.* with modifications made in the Department of Pharmacology and Toxicology. The blood samples of every period were received in glass tubes with sodium citrate as an anticoagulant. The sample treatment technique was by precipitation, whereas the separation one was by high-performance liquid chromatography (HPLC) using detection by Mass Spectrometry.

The pharmacokinetic parameters were determined through non-compartmental methods (independent model). The descriptive statistics are presented below:

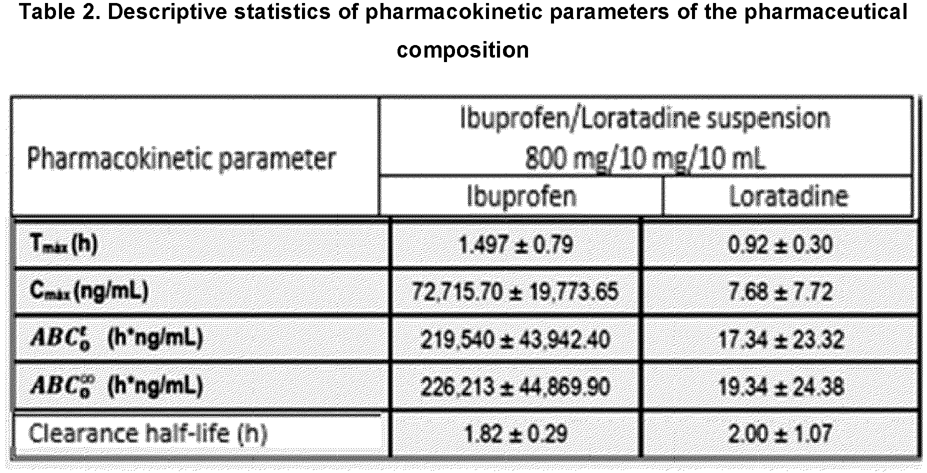

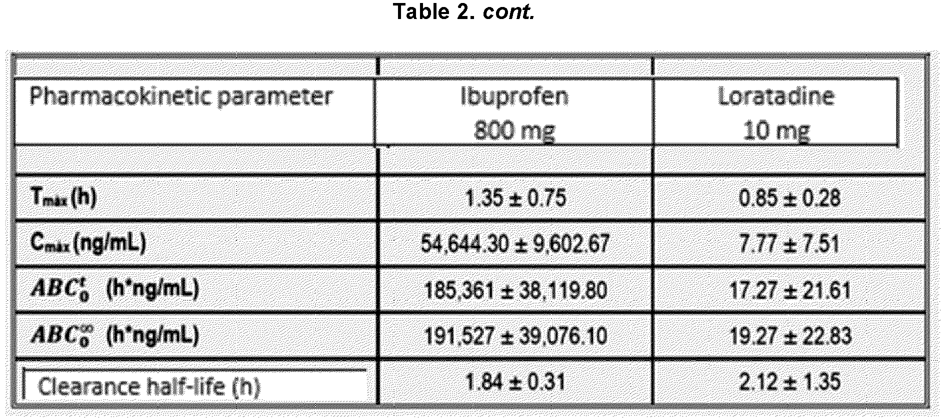

The obtained results indicate that the amount of ibuprofen absorbed (AUC_{t 0} and AUC _{∞ 0}), was similar for the test and reference drug in the established range of 80-125%. For loratadine, the geometric mean ratio for both Cₘₐₓ and AUC_{t 0} and AUC_{∞ 0} are within the set range of 80-125%, therefore establishing that the rate and extent of absorption are similar between the test drug and reference drug. The above, together with the fact that no adverse effects occurred during the study, gives certainty that this product with this dosage form will have a good safety profile in patients.

### Example 4. Stability Tests

### 5 mg / 400 mg Loratadine / Ibuprofen in 5 mL (suspension)

### Stability study results (40°C ± 2°C / 75% RH ± 5% RH)

**Table 3. Product description results**

| **Specifications** | | Initial | 1 month | 3 months | 6 months |
|---|---|---|---|---|---|
| Amber polyethyleneterephthalate (PET) bottle with childresistant cap, containing a homogeneous pink suspension with characteristic odor and flavor, free of lumps and foreign particles. | Batch 1 | Complies | Complies | Complies | Complies |
| | Batch 2 | Complies | Complies | Complies | Complies |
| | Batch 3 | Complies | Complies | Complies | Complies |

**Table 4. Loratadine content results**

| **Specifications** | | **Initial** | **1 month** | **3 months** | **6 months** |
|---|---|---|---|---|---|
| 90.0% to 110.0% | Batch 1 | 100.0% | 100.9% | 100.2% | 101.3% |
| | Batch 2 | 101.6% | 99.3% | 101.8% | 101.2% |
| | Batch 3 | 102.3% | 101.4% | 99.2% | 101.9% |

**Table 5. Ibuprofen content results**

| **Specifications** | | **Initial** | **1 month** | **3 months** | **6 months** |
|---|---|---|---|---|---|
| 90.0% to 110.0% | Batch 1 | 96.9% | 96.9% | 94.6% | 98.9% |
| | Batch 2 | 97.5% | 95.2% | 95.9% | 95.0% |
| | Batch 3 | 98.4% | 95.8% | 96.7% | 96.4% |

**Table 6. Results for loratadine related substances**

| **Specifications** | | **Initial** | **1 month** | **3 months** | **6 months** |
|---|---|---|---|---|---|
| Not more than 0.30% of each unknown individual impurity | Batch 1 | 0.03% | 0.03% | 0.05% | 0.11% |
| | Batch 2 | 0.02% | 0.04% | 0.05% | 0.07% |
| | Batch 3 | 0.03% | 0.04% | 0.05% | 0.06% |
| Not more than 0.50% of total impurities | Batch 1 | 0.07% | 0.03% | 0.1096 | 0.24% |
| | Batch 2 | 0.07% | 0.05% | 0.08% | 0.18% |
| | Batch 3 | 0.08% | 0.04% | 0.08% | 0.19% |

**Table 7. Results for ibuprofen related substances**

| **Specifications** | | **Initial** | **1 month** | **3 months** | **6 months** |
|---|---|---|---|---|---|
| Not more than 0.25% of the related compound C of Ibuprofen | **Batch 1** | 0.00% | 0.01% | 0.00% | 0.01% |
| | **Batch 2** | 0.00% | 0.01% | 0.00% | 0.02% |
| | **Batch 3** | 0.00% | 0.01% | 0.00% | 0.02% |
| Not more than 0.25% of the related compound J of Ibuprofen | **Batch 1** | 0.00% | 0.00% | 0.00% | 0.00% |
| | **Batch 2** | 0.00% | 0.00% | 0.00% | 0.01% |
| | **Batch 3** | 0.00% | 0.00% | 0.00% | 0.01% |
| Not more than 3.50% of the known impurity I of Ibuprofen | **Batch 1** | 0.02% | 0.49% | 1.08% | 2.09% |
| | **Batch 2** | 0.02% | 0.52% | 1.17% | 2.3896 |
| | **Batch 3** | 0.02% | 0.46% | 1.11% | 2.12% |
| Not more than 0.20% of each unknown individual impurity of Ibuprofen | **Batch 1** | 0.02% | 0.08% | 0.02% | 0.07% |
| | **Batch 2** | 0.03% | 0.09% | 0.03% | 0.08% |
| | **Batch 3** | 0.02% | 0.05% | 0.03% | 0.07% |
| Not more than 0.90% of total impurities of Ibuprofen (Without considering the known impurity I of Ibuprofen) | **Batch 1** | 0.07% | 0.12% | 0.11% | 0.21% |
| | **Batch 2** | 0.07% | 0.14% | 0.12% | 0.30% |
| | **Batch 3** | 0.07% | 0.10% | 0.13% | 0.22% |

### Advantages and industrial applications of the invention

This invention relates to stable pharmaceutical compositions of immediate release with improved bioavailability in one dosage unit against respiratory diseases characterized by an acceptable pharmaceutical and therapeutic amount from: a) an analgesic in a concentration of 8 g per 100 mL or an equivalent concentration; b) an antihistamine in a concentration of 0.1 g per 100 mL or an equivalent concentration; c) and one or more pharmaceutically acceptable excipients. The analgesic is selected from ibuprofen or a pharmaceutically accepted salt thereof, while the antihistaminic is selected from loratadine or a pharmaceutically acceptable salt thereof.

Pharmaceutically acceptable excipients are selected from one or more suspending agents, one or more solubilizers, one or more co-solvents, one or more sweeteners or flavorings, one or more colorants, one or more preservatives or antimicrobial agents, and one or more solvents.

In one form, the pharmaceutical composition is adapted to be administered orally in the form of a solution, suspension, emulsion, syrup and/or elixir, or any other pharmaceutically acceptable liquid form, preferably in the form of a suspension. The advantage of the dosage form suspension is that it allows dose adjustment based on medical requirement.

The process of the composition manages to overcome the technological complexity that lies in having both drugs in liquid form preserving the physicochemical properties of the drug substance, as well as conferring on the final product organoleptic properties that guarantee acceptance by the patient.

The pharmaceutical composition for the manufacture of a drug product is useful in the treatment and control of respiratory diseases and acute upper respiratory tract infections (IAVRS) such as acute otitis media, tonsillitis and pharyngogillitis, adenoiditis, rhinopharyngitis or common cold, or allergic rhinitis.

### References

- Amapola Adell Gra, et al., Use of NSAIDs in upper respiratory tract infections; Mexican Journal of Pediatrics, 77(Supp 1), pp S9-S14, 2010
- Benedi, J., Farmacia Profesional, Vol. 19. No. 3. Pages 54-61 (March 2005**)**
- Clissold, S.P., Sorkin, E.M. & Goa, K.L. Loratadine. Drugs 37, 42-57 (1989**)**
- Gwaltney JM, Jr., Winther B, Patrie JT, Hendley JO. Combined antiviral-antimediator treatment for the common cold. J Infect Dis. 2002;186(2):147-54.
- H. Song, R. Zhang, C. Wei, G. Yuan, X. Liu, R. Li, B. Wang, R. Guo Pharmacokinetic and Bioequivalence Studies of Ibuprofen Suspension after a Single-dose Administration in Healthy Chinese Volunteers. Drug Res 2013; 63:383-387.
- Ismaels Ramirez Villaseñor. Use of antimicrobials in acute upper respiratory infections. Rev Med IMSS. 2005:247-55.
- Larenas-Linnemann D, Mayorga-Butron JL, Sanchez-Gonzalez A, Ramirez-Garcia A, Medina-Avalos M, Figueroa-Morales MA, et al. [ARIA Mexico 2014. Adaptation of the Clinical Practice Guide ARIA 2010 for Mexico. Methodology ADAPTE]. Rev Alerg Mex. 2014;61 Suppl 1: S3-S116.
- Llor, C.; BMJ 2013; 347: f5762
- Michael D. Seidman. Clinical Practice Guideline: Allergic Rhinitis Executive Summary. 2015.
- Mirta Alvarez Castelló ea. Recurrent upper respiratory infections. Some considerations. 2008.
- Mossad SB. Current and future therapeutic approaches to the common cold. Expert Rev Anti Infect Ther. 2003;1(4):619-26
- R. Piñeiro Pérez. Consensus document on the diagnosis and treatment of acute pharyngotonsillitis. Pediatrics Yearbooks. 2011
- Ministry of Health. Diagnosis and management of ACUTE UPPER AIRWAY INFECTIONS in patients aged over 3 months up to 18 years. 2016.
- Vlase L, Imre S, Muntean D, Leucuta SE. Determination of loratadine and its active metabolite in human plasma by high-performance liquid chromatography with mass spectrometry detection. Journal of pharmaceutical and biomedical analysis. 2007;44(3):652-7.
- Winther, B.; Infammopharmacology, Vol. 11, No. 4-6, pp. 445-452 (2003**)**

## Claims

1. This is an immediate-release pharmaceutical composition against respiratory diseases that comprises a pharmaceutically and therapeutically acceptable amount of:
a) an analgesic in a concentration of 8 g per 100 ml or an equivalent concentration,
b) an antihistaminic in a concentration of 0.1 g per 100 ml or an equivalent concentration,
c) and one or more pharmaceutically acceptable excipients.

2. As per claim 1, the analgesic of this pharmaceutical composition is selected from ibuprofen base, racemic ibuprofen or its enantiomers, and pharmaceutically acceptable salts thereof.

3. As per claim 1, the antihistamine of this pharmaceutical composition is selected from loratadine or a pharmaceutically acceptable salt thereof.

4. per claims 1 and 2, the ibuprofen of this pharmaceutical composition is in an equivalent concentration of 400 mg in 5 mL.

5. As per claims 1 or 3, the loratadine of this pharmaceutical composition is found in an amount of 5 mg in 5 mL.

6. As per claim 1, the pharmaceutically acceptable excipient of this pharmaceutical composition is selected from one or more suspending agents, one or more solubilizers, one or more co-solvents, one or more sweeteners or flavorings, one or more colorants, one or more preservatives or antimicrobial agents, and one or more solvents.

7. As per claim 6, the suspending agent of this pharmaceutical composition is selected from: carboxymethylcellulose, hydroxypropyl methylcellulose, hydroxypropylcellulose, xanthan gum, tragacanth, bentonite, methylcellulose, polyethylene glycols, microcrystalline cellulose, and carbomer in a concentration of less than 1%.

8. As per claim 7, the suspending agent of this pharmaceutical composition is preferably in a concentration of 0.42%.

9. As per claim 6, the solubilizer of this pharmaceutical composition is selected from ethanol, water, sorbitol, glycerin, polyglycols such as polyethylene glycol (degrees 200 to 6000), polyvinylpyrrolidone (degrees 25, 30, 60, 0 90), polysorbate 20 or polysorbate 80, poloxamer, poloxamer 188, gum arabic, sodium lauryl sulfate, or sodium dioctyl sulfosuccinate, among others, in a concentration greater than 5%.

10. As per claim 9, the solubilizer of this pharmaceutical composition is preferably in a concentration of 6.94%.

11. As per claim 6, one or more co-solvents of this pharmaceutical composition are selected from ethanol, propylene glycol, glycerin, cremophor, polyethylene glycol, polyethylene glycol 300 or 400, and glycerin, or a combination thereof.

12. As per claim 11, the solubilizer of this pharmaceutical composition is preferably in a concentration of 20% Polyethylene glycol and 60% glycerine.

13. As per claim 6, the sweetener or flavorant of this pharmaceutical composition is selected from sucralose, sucrose or fructose, aspartame or acesulfame potassium, and sorbitol, or a combination thereof, in a concentration of 0.42%.

14. As per claim 6, the dye of this pharmaceutical composition is red color No. 40.

15. As per claim 6, the preservative or antimicrobial agent of this pharmaceutical composition agents are selected from ethanol, propanol, betaines, benzalkonium chloride, benzethonium chloride, sodium benzoate, lauric arginate, methylbenzethonium chloride, cetylpyridinium chloride, 2,4,4'-trichloro-2-hydroxy-diphenyl ether (Triclosan), propylparaben, methylparaben, sodium propylparaben, sodium methylparaben, hydroxybutylanisole, chlorhexidine isethionate, chlorhexidine hydrochloride, hexetidine, Quaternium 15, trichlorocarbon, polyhexamethylenebiguanide, cetylpyridinium chloride, imidazolidinylurea, diazolidinylurea, 3-lodo-2-propynyl N-butylcarbamate, 2-methyl-4-isothiazolin-3-one, dimethyldichlorohydricanthine 5-Chloro-2-(2,4-dichlorophenoxy), phenol, glycerol monolaurate, or a combination thereof in a concentration less than 1% .

16. As per claim 6, the solvent of this pharmaceutical composition is preferably water in a pharmaceutically acceptable amount.

17. As per any of the preceding claims, this pharmaceutical composition is adapted to be administered orally in the form of a solution, suspension, emulsion, syrup and/or elixir.

18. As per claim 17, this pharmaceutical composition is in the form of a suspension.

19. As per claims 1 to 18 for the manufacture of a drug product, this pharmaceutical composition is useful in the treatment and control of respiratory diseases and acute upper respiratory tract infections (IAVRS).

20. As per claim 19, the IAVRSs treated with this pharmaceutical composition are selected from acute otitis media, tonsillitis and pharyngogillitis, adenoiditis, rhinopharyngitis or common cold, and allergic rhinitis.
